# EUROPÄISCHE PATENTSCHRIFT

(11) **EP 2 062 224 B1**
(45) Veröffentlichungstag und Bekanntmachung des Hinweises auf die Patenterteilung: **04.08.2010**
(21) Anmeldenummer: 07818105.4
(22) Anmeldetag: 11.09.2007
(51) Int. Cl.: G06T 17/00, A61B 17/66, A61B 17/00, A61B 17/64, A61C 9/00

(54) **VERFAHREN ZUR HERSTELLUNG VON MINDESTENS EINEM OPERATIONSSPLINT, INSBESONDERE FÜR COMPUTERASSISTIERTE MAXILLOFAZIALE OPERATIONEN UND UMSTELLUNGSOSTEOTOMIEN**
METHOD FOR PRODUCTION OF AT LEAST ONE SURGICAL SPLINT, IN PARTICULAR FOR COMPUTER-ASSISTED MAXILLOFACIAL OPERATIONS AND TRANSPOSITION OSTEOTOMIES
PROCÉDÉ DE FABRICATION D'AU MOINS UNE GOUPILLE CHIRURGICALE FENDUE, EN PARTICULIER POUR LA CHIRURGIE MAXILLOFACIALE ET L'OSTÉOTOMIE DE TRANSFORMATION ASSISTÉES PAR ORDINATEUR

(30) Priorität: 11.09.2006 DE 102006043204
(43) Veröffentlichungstag der Anmeldung: 27.05.2009
(73) Patentinhaber: Zinser, Jochen Max, 50678 Köln (DE); Zöller, Joachim E., 69126 Heidelberg (DE)
(72) Erfinder: Zinser, Jochen Max, 50678 Köln (DE); Zöller, Joachim E., 69126 Heidelberg (DE)
(74) Vertreter: Maxton Langmaack & Partner
(86) Internationale Anmeldenummer: PCT/EP2007/007907
(87) Internationale Veröffentlichungsnummer: WO 2008/031562

(56) Entgegenhaltungen:
- EP-A- 1 502 556
- WO-A-2006/000063
- US-A1- 2003 138 755
- US-B1- 6 254 639
- US-B1- 6 547 796
- TROULIS M J ET AL: "DEVELOPMENT OF A THREE-DIMENSIONAL TREATMENT PLANNING SYSTEM BASED ON COMPUTED TOMOGRAPHIC DATA" INTERNATIONAL JOURNAL OF ORAL AND MAXILLOFACIAL SURGERY, COPENHAGEN, DK, Bd. 31, Nr. 4, August 2002 (2002-08), Seiten 349-357, XP008053607 ISSN: 0901-5027

## Beschreibung

Die vorliegende Erfindung betrifft ein Verfahren zur Herstellung von mindestens einem Operationssplint, insbesondere für computerassistierte maxillofaziale Operationen und Umstellungsosteotomien, sowie mittels dieses Verfahrens hergestellte Operationssplinte. Das Verfahren ist dabei jedoch nicht auf die vorstehend angegebenen beispielhaften kieferchirurgischen Gebiete beschränkt, sondern kann für die Herstellung jeglicher Art von Splinten im Schädelbereich eingesetzt werden.

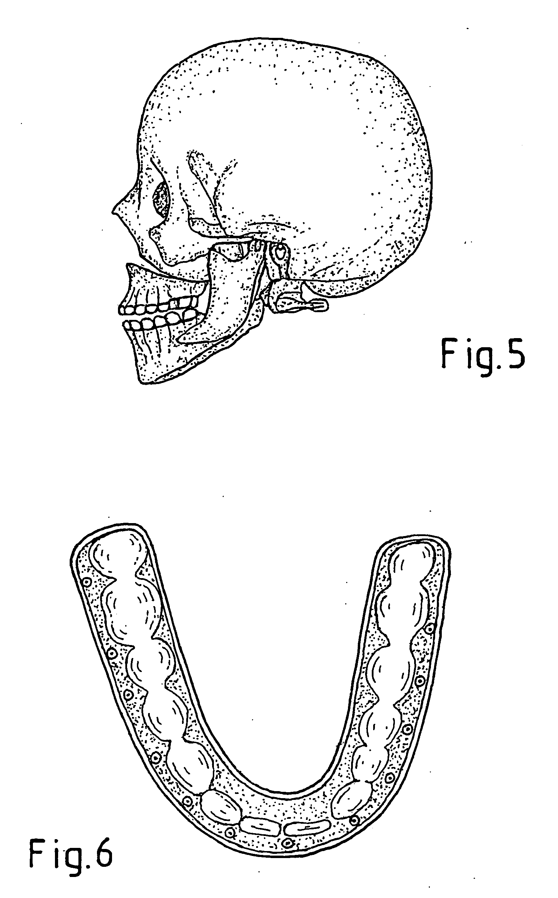

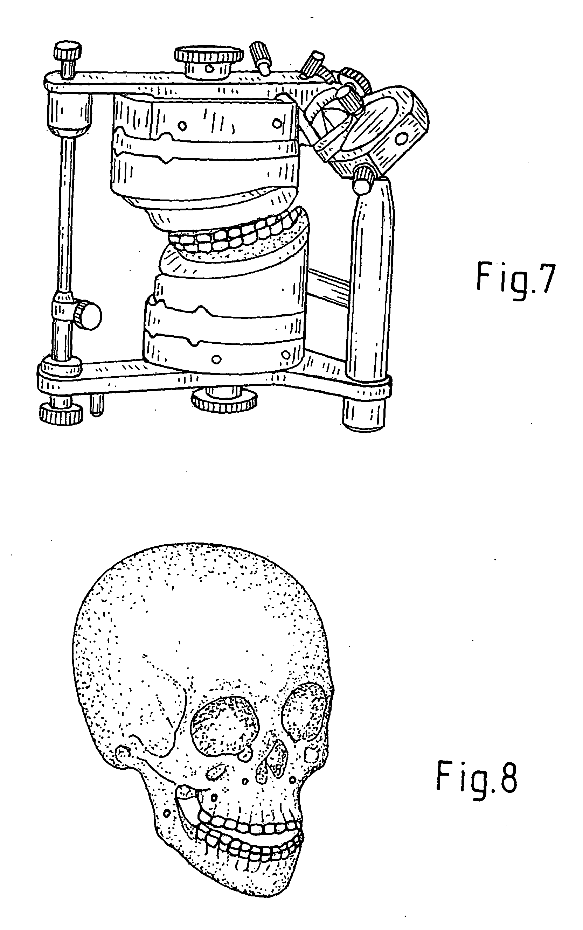

Traditionell werden bei der Planung von orthognathen-maxillofazialen Operationen mehrere Diagnoseverfahren miteinander kombiniert. Anthropometrische und zephalometrischen Messungen sowie ästhetische Richtlinie tragen zur Planung bei und bilden die Grundlage für die metrische Analyse. Ein wesentlicher Bestandteil der Operationsplanung sind die Gipsabformungen der Zähne beziehungsweise der Abformung im Ober- und Unterkiefer. Des Weiteren besteht die Möglichkeit, anhand von Fotografien eine Weichteilsimulation durchzuführen. Diese diagnostischen Methoden ermöglichen es dem Kliniker, chirurgische Bewegungen der Kiefersegmente und Zähne durchzuführen. Um die Behandlungspläne chirurgisch im Operationssaal umzusetzen, müssen Splinte beziehungsweise chirurgische Schienen (siehe Fig. 6) hergestellt werden, welche die Information, das heißt die räumliche Lagebeziehung des Ober- und Unterkiefers in Relation zum Schädel umsetzen. Hierfür werden traditionell die Gipsmodelle des Oberkiefers und Unterkiefers mittels einer Gesichtsbogenregistrierung in den Artikulator (siehe Fig. 7) übertragen. Nun kann die räumliche Lageveränderung der Kiefer, das heißt die neue Planung vorgenommen werden. Als Grundlage dienen die oben beschriebenen Verfahren zur metrischen Analyse. Durch die Verschiebung der Kiefer im Artikulator entsteht die neue gewünschte Lageposition der Kiefer. Die Zahnabstützung, beziehungsweise die Kontaktpunkte oder Lagebeziehung von Ober- und Unterkiefer werden durch die Gipsmodelle bestimmt.

In der Regel werden bei bimaxillären Umstellungsosteotomien zwei Operationssplinte benötigt. Ein Zwischensplint repräsentiert die neue Position des Oberkiefers und die alte Position des Unterkiefers. Dieser wird eingesetzt, nachdem der Oberkiefer knöchern abgetrennt und mobilisiert wurde, um die neue gewünschte räumliche Position des Oberkiefers festzulegen. Nach knöcherner Fixation des Oberkiefers mit Platten wird der Unterkiefer gespalten und mobilisiert. Die Position des Unterkiefers wird in der Regel durch die Bezahnung beziehungsweise dentale Abstützung des Oberkiefers bestimmt. Ein Endsplint wird dann nach Verschiebung des Unterkiefers oder Adjustierung der Kiefersegmente in der gewünschten Position im Artikulator hergestellt. Bei monomaxillären Umstellungsosteotomien wird nur ein Splint benötigt. Dieser wird in gleicher Art und Weise im Artikulator hergestellt und richtet sich nach der Bezahnung und Lage des Gegenkiefers.

Die Nachteile der traditionellen Verfahren sind folgende:
1. Die oben beschriebene Diagnostik bezieht die dreidimensionalen Knochenstrukturen des Schädels nicht mit ein. Im speziellen sind schiefe Schädelkonfigurationen und knöcherne Missbildungen nur schwer zu erfassen und metrisch auszuwerten. Sämtliche cephalometrische Analysen basieren auf zweidimensionaler Darstellung mit begrenzter Aussagekraft bei fehlender Dreidimensionalität.
2. Die intraoperative Übertragung der im Artikulator geplanten neuen Kieferposition ist nur eingeschränkt möglich, sie umfasst nur die zahntragenden Knochensegmente, die in den Splint miteinbezogen sind. Sämtliche distal der Osteotomielinie liegenden Knochenteile können durch die Splinte nicht erfasst werden. Da das Kiefergelenk ein mobiles flexibles Gelenk ist, ist eine rigide Abstützung nicht möglich. Im speziellen ist es nahezu ausgeschlossen vor allem bei bimaxillären Umstellungsosteotomien durch die Eigenbeweglichkeit des Unterkiefers im Kiefergelenk eine metrisch präzise Lagebeziehung des Knochensegmente (Oberkiefer) in der gewünschten Position herzustellen.
3. Zudem verändert sich die Position des aufsteigenden gelenktragenden Kieferastes nach der sagittalen chirurgischen Spaltung. Das abgetrennte distale hinter der Osteotomielinie liegende Kiefersegment ist frei beweglich. Entsprechend kann die präoperative Ausgangslage oder Kiefer-Kiefergelenksbeziehung mit den traditionellen Splinten nicht wiederhergestellt werden. Die herkömmlichen Operationssplinte ermöglichen zwar eine optimale Zahn-zu-Zahnabstützung im anterioren (vorderen) Kieferbereich, können jedoch die ursprüngliche Kiefergelenksbeziehung nicht wiederherstellen. Dadurch können langfristig Kiefergelenksbeschwerden entstehen.

In zahlreichen Weiterentwicklungen, bzw. Arbeiten wurde versucht, die oben beschriebenen Nachteile zu verbessern. Um die knöcherne Gesamtsituation des Schädels in die Planung mit einzubeziehen, wurden zum Beispiel Stereolithografiemodelle hergestellt, um eine räumliche dreidimensionale Diagnostik durchzuführen. Dieses Verfahren ist sehr teuer und umständlich.

Des Weiteren wurden Verfahren entwickelt, die anhand von DVT, CT und MRT Daten eine dreidimensionale Diagnostik und Operationsplanungen unter Berücksichtigung des gesamten Schädels ermöglichen. Diese Softwaremodule oder Planungseinheiten können gemäß WO 2006/000063 A1 (Basis für den Oberbegriff des Anspruchs 1) als Planungseinheit für orthognathe oder maxillofaziale Fragestellungen genutzt werden. Zudem wurden Verfahren beschrieben, welche anhand der virtuellen Operationsplanungen direkt chirurgische Operationssplinte herstellen. Die Operationssplinte gleichen den traditionellen Okklusionssplinten und beinhalten das gleiche Funktionsprinzip. Die Herstellung dieser Splinte basiert auf dem Stereolithografieverfahren oder einer ähnlichen Technik (siehe beispielsweise WO 03/028577 A2).

Die WO 03/0208577 A2 bezweckt die Lösung des Problems der Erzielung einer definierten Zahnstellung eines Patienten bei einer kieferchirurgischen Operation. Hierzu werden in einem Computermodell Markierungspunkte, welche aus einem Gipsmodell, erhalten aus einem Abdruck des Gebisses des Patienten, ermittelt wurden und in einer Ebene mit der Okklusionsebene liegen, übertragen, und das Computermodell mit Hilfe dieser Markierungspunkte dem Gipsmodell, welches die tatsächliche Zahnstellung des Patienten wiedergibt, angeglichen. Diese Markierungspunkte oder "fiducial marcers" sind dabei stiftartig oder kugelförmig ausgebildet und sind vorzugsweise aus Titan, da dadurch keine Artefakte entstehen gefertigt. Die Markierungspunkte dienen also dazu, die Relation der beiden Kiefer im Speziellen die im Gipsmodell nicht verzerrten Oberflächen der Zähne in den 3-D-Datensatz zu matchen. Entsprechend entsteht dadurch eine artgetreue Abbildung der Zähne die nicht durch Artefakte oder Streustrahlung verändert werden. Anhand der eingescannten und gematchten Gipsmodelle werden dann die Operationssplinte hergestellt. Nachfolgend werden diese Markierungspunkte oder "fiducial markers" dann aus dem Datensatz entfernt, bzw. nicht weiter berücksichtigt. Ein Zusammenhang oder Verwendung der Marker in der Planungs- oder Umsetzungsphase der computer assistierten Planung besteht nicht.

Mittels dem in der WO 2006/000063 A1 offenbarten Analyseverfahren können jeweils U-förmige Splinte betreffend die dentale Okklusionsebene hergestellt werden. Auch gemäß dem dort offenbarten Verfahren wird wiederum anhand von Gipsmodelldaten dreidimensionale, mit einer Software erzeugte Daten in Konkurrenz gebracht, wobei dann nachfolgend ausschließlich im Hinblick auf die dentale Okklusionsebene ein 2D-Operationssplint erzeugt wird. Dieser 2D-Operationssplint wird zwar mittels computer-assistierter Chirurgie geplant und hergestellt. Jedoch ist die Verwendung durch das 2-D-Design limitiert, eine direkte Umsetzung der virtuellen Planung in den operativen Situs ist nicht möglich. Vom Konzept her müssen die zu bewegenden Knochenteile, z.B. der Oberkiefer trotz computer assistierter Planung, zunächst am Unterkiefer orientiert werden um die neue, gewünschte Endposition der Planung zu erhalten. Also ist mit dem 2-D-Operationssplint eine direkte unabhängige Positionierung oder direkte Umsetzung der virtuellen Operationsplanung nicht möglich. Auch mit dem in der WO 2006/000063 A1 offenbarten Verfahren ist es wiederum nicht möglich, insbesondere Fehlstellungen der Kiefergelenksposition zu berücksichtigen, bzw. die zentrische Gelenksposition des Unterkiefers zu gewährleisten.

Trotz der beschriebenen Verfahren und Verbesserungen bestehen weiterhin die bereits oben unter 2. und 3. beschriebenen Nachteile:
- Die dort offenbarten chirurgischen Splinte ähneln vom Funktionsprinzip den traditionellen zweidimensionalen chirurgischen Splinten. Eine exakte und präzise metrische Übertragung der virtuellen computerassistierten Planung, das heißt, der räumlichen Position der geplanten Knochenstücke in den operativen Situs mittels der chirurgischen Splinte ist mit diesen zweidimensionalen Splinten nicht möglich.
- Wie oben bereits beschrieben verändert sich die Kiefergelenksposition der Kiefergelenke durch die Operation. Eine exakte Reposition ist auch mit den verbesserten "traditionellen" Splinten nicht möglich.

Die Aufgabe der Erfindung ist es daher, die oben genannten Probleme zu lösen, beziehungsweise Verbesserungen zu erzielen.

Diese Aufgabe wird durch das Verfahren gemäß den Ansprüchen als auch durch den Splint gemäß Anspruch 13 gelöst. Weitere vorteilhafte Ausbildungen können den Unteransprüchen entnommen werden.

Das erfindungsgemäß Herstellungsverfahren ermöglicht somit aufgrund einer digitalen Planung eines zunächst dreidimensionalen virtuellen Splintes die Herstellung eines tatsächlichen Splintes, wobei die Herstellung beispielsweise mit Rapidprototyping anhand der im Computer mittels der Software generierten Daten erfolgen kann. Insbesondere können mit dem erfindungsgemäßen Verfahren auch mehrere verschiedene dreidimensionale Operationssplinte, je nach Anforderung der Operation, erzeugt werden. Dabei kann auch ein so genannter Referenzierungssplint erzeugt werden, welcher mindestens drei Referenzierungspunkte oberhalb bzw. gegenüber einer Osteotomielinie eines zu bewegenden Knochensegmentes, d.h. insbesondere cranial, aber auch an jeder anderen Position, aufweist, wobei dieser Referenzierungssplint quasi als Schablone dient. Die Referenzierungspunkte können beispielsweise auch ohne Einsatz der Software metrisch oder auf sonstige Art und Weise festgelegt werden. Die Gelenkposition des Kiefers wird vorteilhafterweise beibehalten.

Mit dem erfindungsgemäßen Herstellungsverfahren ist es möglich, eine exakte räumliche Lagebeziehung segmentierter Knochenstücke beziehungsweise des Ober- und Unterkiefers zum Schädel herzustellen. Hierdurch werden Artefakte des Schädelbaus und des Kiefers beim Patienten berücksichtigt, so dass die einleitend beschriebenen Nachteile nicht auftreten. Insbesondere werden die Kiefergelenke in Ausgangsposition gehalten, und werden durch das Herstellungsverfahren nicht lageverändert, beziehungsweise deren Position wird durch die Auswahl von drei Referenzierungspunkten an definierten Schädelteilen und/oder in frei wählbaren Bereichen mindestens eines ersten Knochensegmentes oberhalb bzw. gegenüber einer Osteotomielinie eines zu bewegenden Knochensegmentes definiert.

Die Erzeugung dreidimensionaler Daten kann insbesondere mit Hilfe von Röntgenstrahlbasierten Verfahren erfolgen, beispielsweise können Hardgewebs- als auch Weichgewebsdatensätze ermittelt werden auf Grundlage von CT, DVT und MRT-Datensätzen des Schädels. Auf Grundlage derart ermittelter Datensätze ist es möglich, mittels dem erfindungsgemäßen Herstellungsverfahren Operationssplinte zu erzeugen, welche Artefakte eines Patienten insbesondere im Kieferbereich, aber auch in anderen Bereichen des Schädels berücksichtigen. Mittels dem neuartigen Herstellungsverfahren ist es ermöglicht, virtuelle Operationsplanungen eines Computers direkt auf den operativen Situs umzusetzen und die Planung in situ zu transformieren.

Das erfindungsgemäße Herstellungsverfahren ermöglicht es insbesondere, Planungsdaten des Computers, das heißt die räumliche Position von Knochensegmenten, einschließlich Knochenstücken, zahntragenden Knochensegmenten beziehungsweise der Kiefer, präzise intraoperativ umzusetzen. Im Speziellen gelingt es, die segmentierten Knochenstücke an der gewünschte Stelle anhand der erfindungsgemäßen Splinte in situ zu platzieren.

Das erfindungsgemäße Verfahren zeichnet sich insbesondere dadurch aus, dass die ursprüngliche Position der Kiefergelenke, das heißt die präoperative Position, durch den operativen Eingriff nicht verändert wird, beziehungsweise die präoperative Situation wieder hergestellt werden kann. Ursächlich hierfür sind die eben mittels des erfindungsgemäßen Herstellungsverfahrens hergestellten dreidimensionalen Operationssplinte. Dadurch können Kiefergelenksfolgeerkrankungen vermieden werden. Dabei ist die Konzeption der Splinte unabhängig von der Anzahl der zu verschiebenden Knochenteile, das heißt bei bimaxilären Umstellungsosteotomien können insgesamt bis zu drei Splinten und bei monomaxilären Umstellungsosteotomien können bis zu zwei Splinten vorgesehen sein. Bei bimaxilären Umstellungsosteotomien kann dabei ein Referenzierungssplint auch als üblicher chirurgischer Splint beziehungsweise aufgrund metrischer Daten erstellt werden. Die Splinte können anhand der mit dem erfindungsgemäßen Herstellungsverfahren ermittelten Daten für die virtuellen Splinte direkt als Formteile mit geeigneten Verfahren hergestellt werden.

Durch das erfindungsgemäße Herstellungsverfahren lassen sich die zu verschiebenden Knochensegmente, einschließlich Knochenstücke, zahntragenden Kiefersegmente und Kiefer, intraoperativ anhand der als Formteile hergestellten Splinte in die gewünschte Lage verschieben und fixieren.

Die erfindungsgemäß hergestellten Splinte erlauben es dabei, dass die präoperative Position der Kiefergelenke durch eine Operation, das heißt die Umstellungsosteotomie, nicht verändert wird und operativ wiederhergestellt wird. Dabei wird durch die Bauweise der erfindungsgemäßen dreidimensionalen Splinte die ursprüngliche Kiefergelenksposition berücksichtigt und wiederhergestellt.

Durch die Auswahl dreier Referenzierungspunkte oberhalb bzw. gegenüber einer Osteotomielinie eines zu bewegenden Knochensegmentes erfolgt eine dreidimensionale Definition und Festlegung der Position der Schädelteile und/oder ersten Knochensegmente, auf welche die Referenzierungspunkte bezogen sind, zu dem zu verschiebenden zweiten Knochensegment. Durch diese vorherige Definition der räumlichen Lage durch Wahl der Referenzierungspunkte an einer Position oberhalb der Osteotomielinie ist es ermöglicht, nachfolgend der räumlichen Ausrichtung des zweiten Knochensegmentes und Auswahl von mindestens einem Verbindungspunkt auf einer Okklusionsplatte einen dreidimensionalen, zunächst virtuellen Splint durch Ausbildung von Verbindungsarmen zwischen eben diesem mindestens einem Verbindungspunkt und mindestens einem der drei Referenzierungspunkte zu erzeugen. Die Okklusionsplatte im Sinne der vorliegenden Erfindung entspricht dabei den Zahnkontakten und damit der Okklusionsebene, und dabei den traditionellen Splinten. Da bei zahnlosem Kiefer, wie dieser in den dreidimensionalen Datensätzen in dem erfindungsgemäßen Herstellungsverfahren vorliegen kann, eine Okklusionsebene nicht bestimmbar ist, wird in der vorliegenden Erfindung eben der Begriff Okklusionsplatte verwendet. Erfolgt im Sinne der vorliegenden Erfindung eine räumliche Ausrichtung eines zweiten segmentierten, d.h. des zu bewegenden Knochensegmentes in den dreidimensionalen Datensätzen dahingehend, dass hierdurch die Position des zweiten Knochensegmentes vor der Operation wiedergegeben ist, so kann durch die dann gebildete Okklusionsplatte und mindestens einen dort festgelegten Verbindungspunkt ein Referenzierungssplint durch Verbindung mit sämtlichen der mindestens drei Referenzierungspunkte erhalten werden. Hierdurch wird im Prinzip eine Schablone erzeugt, anhand welcher insbesondere die Kiefergelenksposition genau definiert ist. Wird die räumliche Ausrichtung des zweiten, segmentierten Knochensegmentes hingegen derart vorgenommen, dass beispielsweise das betreffende zweite Knochensegment im Unterschied zu der Position vor einer möglichen Operation und damit im Unterschied zu den zunächst erhaltenen dreidimensionalen Datensätzen verschoben ist, kann durch die Festlegung mindestens eines Verbindungspunktes auf der dann gebildeten neuen Okklusionsplatte mit mindestens zwei Referenzierungspunkten ein zunächst virtueller Operationssplint mit zwei Verbindungsarmen ausgebildet werden, welcher dann beispielsweise mittels Rapidprototyping als dreidimensionales Formteil hergestellt und in der Operation eingesetzt werden kann. Es kann weiterhin auch vorgesehen sein, dass zur Okklusionssicherung und Reposition des Kiefergelenkes ein weiterer Splint, insbesondere Endsplint genannt, zunächst virtuell hergestellt wird durch die Auswahl eines Verbindungspunktes an der durch Verschiebung gebildeten neuen Okklusionsplatte und Verbindung dieses Verbindungspunktes durch Ausbildung eines Verbindungsarmes mit einem weiteren Referenzierungspunkt erfolgt. Dieser weiterer Referenzierungspunkt entspricht dabei einer Repositionsmarkierung, durch welche die ursprüngliche, präoperative Kiefergelenksbeziehung definiert ist. Diese Repositionsmarkierung ist bei dem vorgenannten, durch Verbindung mindestens eines oder zweier Verbindungspunkte mit zwei weiteren Referenzierungspunkten gebildeten Operationssplintes nicht umfasst.

Das erfindungsgemäße Verfahren und die mittels diesem hergestellten erfindungsgemäßen Splinte sind insbesondere bei bimaxilären Umstellungsosteotomien, aber auch bei monomaxilären oder Segmentosteotomien anwendbar beziehungsweise einsetzbar. Entsprechend verändert sich lediglich die Anzahl der Splinte in Abhängigkeit von den zu verschiebenden Knochenstücken auf zum Beispiel nur zwei oder nur einen. Im Sinne der vorliegenden Erfindung kann insbesondere durch die mindestens drei Referenzierungspunkte auch zwei Splinte erzeugt werden, wobei dann einem Splint zwei Referenzierungspunkte, und einem Splint, insbesondere Endsplint, ein (anderer) Referenzierungspunkt zugeordnet wird.

Erfindungsgemäß kann weiterhin vorgesehen sein, dass zwischen Verbindungsarmen des Splintes eine stabilisierende Verstrebung erzeugt wird. Diese kann dann in dem erfindungsgemäß hergestellten Formteil auf Grundlage der dreidimensionalen Datensätze erzeugt werden. Des Weiteren kann vorteilhaft vorgesehen sein, dass der mindestens eine Splint mit mindestens einer Markierung für eine Osteotomielinie versehen ist.

Erfindungsgemäß erfolgt die Verbindung mindestens eines Referenzierungspunktes mit mindestens einem Verbindungspunkt dergestalt, dass jeweils räumlich benachbarte Referenzierungs- und Verbindungspunkte miteinander verbunden werden. Bei Vorsehung von drei Referenzierungspunkten können daher insbesondere auch drei Verbindungspunkte vorgesehen sein. Durch die Auswahl der Verbindungspunkte jeweils auf der Okklusionsplatte ist hierdurch die Ausbildung eines dreidimensionalen Splintes mit innewohnenden definierten räumlichen Parametern ermöglicht, beispielsweise bei der Ausbildung eines Referenzierungssplintes für bimaxiläre Umstellungsosteotomien.

Vorteilhafterweise erfolgt bei dem erfindungsgemäßen Verfahrens zusätzlich die Erstellung eines digitalen Datensatzes der Zähne des Patienten, erhalten über Gipsabdrücke derselben, wobei diese in den dreidimensionalen Datensatz vom Knochen des Schädels einschließlich des Kiefers integriert werden. Dabei kann vorteilhafterweise eine Überlagerung des digitalen Datensatzes der Zähne mit dem Knochendatensatz erfolgen, und auf diese Weise eine bestmögliche Wiedergabe der Zahnformen gewährleistet und Bildgebungsartefakte vermieden werden. Des Weiteren wird hierdurch die genaue Präzision der Zähne erhalten.

Diese und weitere Vorteile der vorliegenden Erfindung werden anhand der nachfolgenden Figuren näher erläutert. Es zeigen:
- Fig. 1:: eine dreidimensionale Darstellung einer skeletalen Anomalie;
- Fig.2:: einen erfindungsgemäßen Referenzierungssplint;
- Fig.3:: einen zweiten erfindungsgemäßen Positionierungs- beziehungsweise Reposi- tionierungssplint;
- Fig.4:: einen dritten Operationssplint, als Endsplint ausgebildet, zur Okklusionssiche- rung und Reposition eines Kiefergelenks;
- Fig.5:: eine dreidimensionale Darstellung der operierten skelletalen Anomalie nach virtueller Planung und Verschiebung der Knochenteile;
- Fig. 6:: Konventioneller "traditioneller" zweidimensionaler Operationssplint nach dem Stand der Technik;
- Fig. 7:: Konventionelle "traditionelle" Operationsplanung im Artikulator;
- Fig. 8:: Referenzierungslöcher in einem Kiefer im Originalzustand, vor der virtuellen Umstellungsoperation, im Oberkiefer für das Oberkiefersegment sowie im Un- terkiefer jeweils eine Markierung für die aufsteigenden Kieferäste rechts und links in einer Schrägansicht;
- Fig. 9:: Referenzierungslöcher in einem Kiefer im Originalzustand, vor der virtuellen Umstellungsoperation, im Oberkiefer für das Oberkiefersegment sowie im Un- terkiefer jeweils eine Markierung für die aufsteigenden Kieferäste rechts und links in einer Vorderansicht;
- Fig. 10:: Referenzierungssplint mitsamt Schädel vor der Osteotomie mit Verbindungs- armen zu den Referenzierungspunkten gemäß Fig. 8 und 9 und einer Markie- rung für die geplante Osteotomielinie;
- Fig. 11:: Positionierungs- beziehungsweise Repositionierungssplint mitsamt Schädel und Positionierung der segmentierten Knochenstücke in einem Oberkiefer- segment, welches nach vorn, anterior verschoben und nach kaudal gekippt wurde, um den offenen Biss zu schließen in einer Schrägansicht;
- Fig. 12:: Positionierungs- beziehungsweise Repositionierungssplint mitsamt Schädel und Positionierung der segmentierten Knochenstücke in einem Oberkiefer- segment, welches nach vorn, anterior verschoben und nach kaudal gekippt wurde, um den offenen Biss zu schließen in einer Seitenansicht; und
- Fig. 13:: eine Schrägansicht eines gemäß dem erfindungsgemäßen Verfahren operier- ten Kiefers mit einem Endsplint, der die Endposition und Kiefergelenksposition definiert.

Zunächst sei vorausgeschickt, dass die in den Figuren gezeigten Merkmale nicht auf die einzelne Ausgestaltung beschränkt sind. Vielmehr sind die jeweils in der Beschreibung einschließlich der Figurenbeschreibung und Zeichnung angegebene Merkmale zur Weiterbildung miteinander kombinierbar. Der Aufbau und das Verfahren zur Herstellung der erfindungsgemäßen Splinte wird im Folgenden am Bespiel einer bimaxilären Umstellungsosteotomie erklärt, wofür insgesamt bis zu drei Splinten hergestellt werden können, nämlich ein Referenzierungssplint, ein Positionierungs- und/oder Repositionierungssplint und ein dritter Splint (Endsplint).

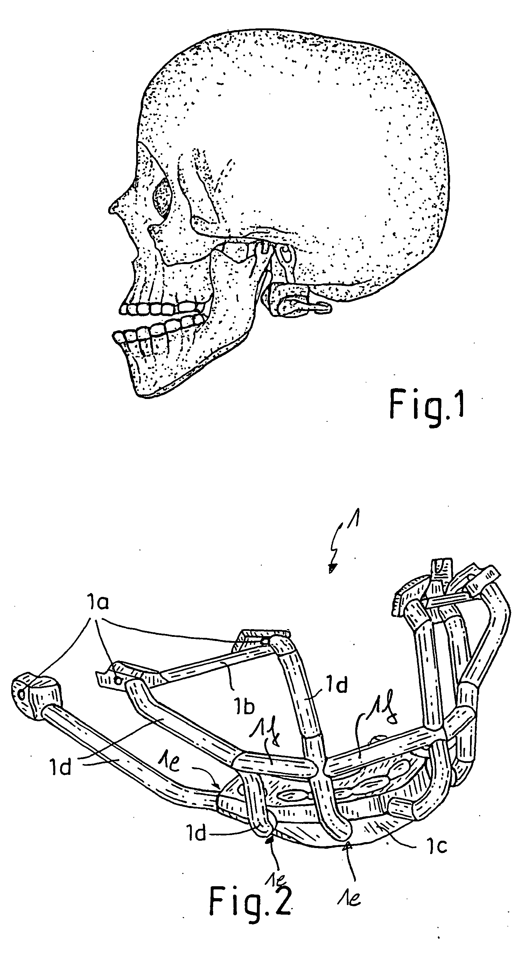

Fig. 1 zeigt eine dreidimensionale Darstellung einer skeletalen Anomalie, nämlich eine Progenie mit offenem Biss, Fig. 5 den operierten Zustand mit einem Oberkiefersegment, welches von vorne anterior verschoben und nach kaudal gekippt wurde, um den offenen Biss zu schließen.

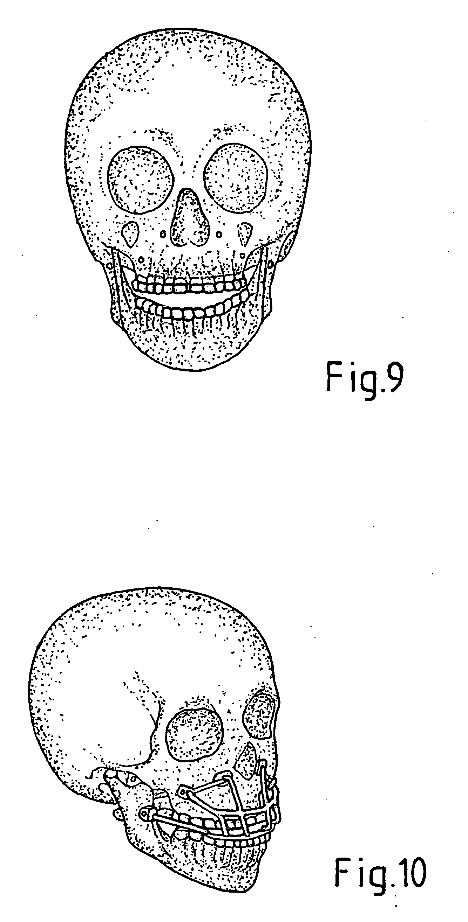

In einem ersten Schritt werden die relevanten Knochendatensätze erstellt durch beispielsweise Computertomografie, wobei insbesondere die zu verschiebenden Knochensegmente und zahntragenden Knochenteile erfasst werden müssen. Auf Grundlage dieser Datensätze wird eine Abbildung der relevanten Knochenteile mittels Software dargestellt. In einem ersten Schritt, das heißt bevor die Knochensegmente, einschließlich zahntragenden Knochenteile oder Kiefer, verschoben werden, müssen oberhalb und cranial der Osteotomielinien Referenzierungspunkte angebracht werden. Dies kann mittels Software dadurch erfolgen, dass in der dreidimensionalen Abbildung Löcher, also Referenzierungslöcher, angeordnet werden. Um eine dreidimensionale Lage eines Knochensegmentes im Raum zu gewährleistet, sind dabei mindestens drei Referenzierungspunkte pro Knochensegment notwendig (siehe hierzu auch Fig. 8 und 9), wobei auf jeder Seite des Kiefers hier zwei im Oberkiefer und eines im Unterkiefer, genauer im ansteigenden Kiefergelenkfortsatz des Unterkiefers, angeordnet sind. Um eine genaue Präzision der Zähne zu erhalten, werden erstellte Gipsmodelle eingescannt (digitalisiert) und mit dem Knochen durch die eingesetzte Software überlagert.

Nunmehr wird aufgrund der hier beschriebenen bimaxilären Umstellungsosteotomie zunächst in Referenzierungssplint 1 (Fig. 2 und 10) angefertigt. Dieser kann jedoch auch beispielsweise einfach aufgrund metrischer Daten erstellt werden, welche als Datensatz der Software zugeleitet werden. In diesem Fall werden die Referenzierungspunkte vorweg metrisch bestimmt und die entsprechenden Daten der Software zugeleitet werden, welche dann an den entsprechenden Positionen die vorerwähnten Referenzierungslöcher erstellt. Der Referenzierungssplint besteht aus einer als Okklusionsebene ausgebildeten Okklusionsplatte 1c, welche einen klassischen zweidimensionalen Operationssplint (siehe hierzu Fig. 6), welcher U-förmig ausgebildet ist, darstellt. Die Okklusionsebene entspricht dabei den Zahnkontakten, und damit den traditionellen Splinten. Die vorliegende Erfindung zeichnet sich dadurch aus, dass mindestens drei Referenzierungslöcher 1a nur mit Verbindungspunkten 1e zur Bildung von Verbindungsarmen 1d verbunden werden, wobei die Verbindungspunkte mittels Software festgelegt beziehungsweise ermittelt werden, und von der Okklusionsebene 1 c ausgehen. Des Weiteren können an dem Splint Markierungen 1b für Osteotomielinien angebracht werden. Dabei können auch Stabilisierungsverstrebungen 1f vorgesehen sein, die quer zu den Verbindungsarmen 1d verlaufen und diese zumindest teilweise verbinden. Der Splint 1 gemäß Fig. 2 weist somit links und rechts des Kiefers jeweils insgesamt drei Referenzierungspunkte 1a auf. Die Referenzierungspunkte beziehungsweise -löcher können dabei mittels Software mit kleinen Auflagen versehen werden, die am Knochen aufliegen. Die Ausbildung von Auflagen ist dabei generell ein jeder Art mittels dem erfindungsgemäßen Verfahren hergestellten Splinten möglich, und erleichtert später den operativen Einsatz der mittels dem erfindungsgemäßen Verfahren als Formteile herstellten Operationssplinte. Die Referenzierungspunkte, welche mittels Software als Referenzierungslöcher ausgebildet sein können, können dabei einen solchen Durchmesser aufweisen, dass bei Einsatz eines Splintes Halteschrauben zur Fixation desselben verwendet werden können. Dies ist insbesondere vorteilhaft bei Einsatz eines Positionierungssplintes.

In einem zweiten Schritt kann nach der in dem ersten Schritt erfolgten Festlegung der Position des Kiefers im dreidimensionalen Raum mittels Software die Operation im Hinblick auf die jeweiligen Gegebenheiten virtuell geplant und die zu verschiebenden und neu zu positionierenden Knochenteile wunschgemäß ausgerichtet und bewegt werden, wobei sichergestellt ist, dass die Kieferstellung bei der Operation erhalten bleibt, und so Kiefergelenksfolgeerkrankungen vermieden werden.

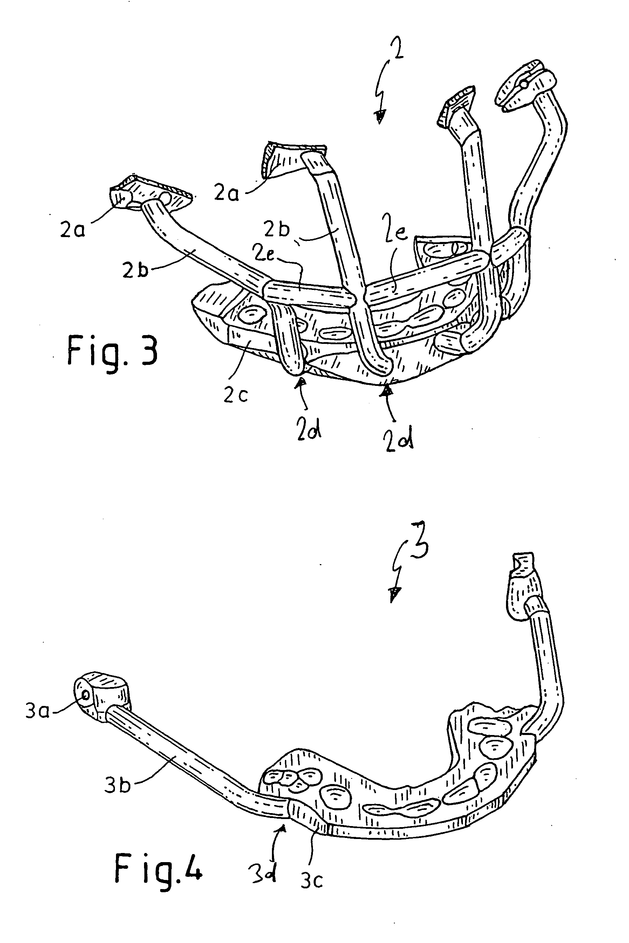

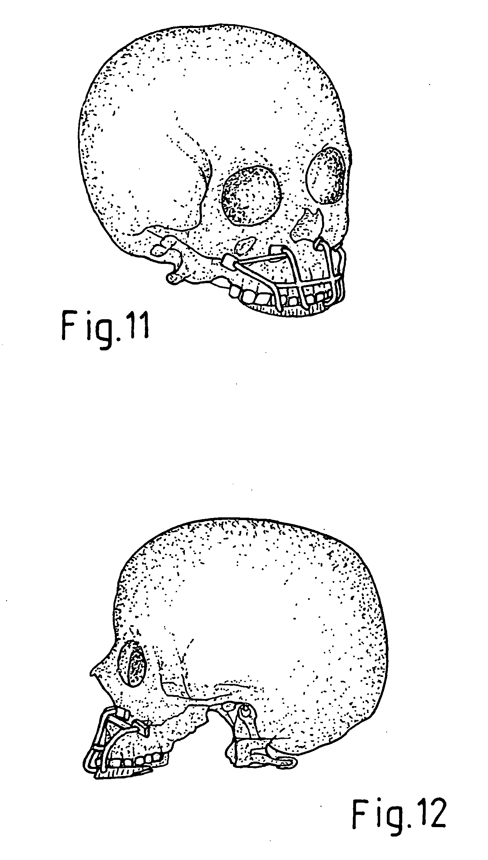

Hierzu wird im zweiten Schritt ein Positionierungs- und Repositionierunssplint 2 (Fig. 3 und 11) hergestellt, welcher ein segmentiertes und somit mobilisiertes zahntragendes Knochenstück (zweites Knochensegment) hält und definiert. Die wesentlichen Merkmale des Referenzierungs- und Repositionierungssplintes 2 sind dabei Folgende:

Die Referenzierungspunkte 2a entsprechend zwei Referenzierungspunkten 1a des Splintes 1 werden wiederum mittels Software als Referenzierungslöcher ausgebildet. Damit wurden die Referenzierungspunkte 2a durch den Referenzierungssplint 1 vor der Verlagerung räumlich definiert. Diese Referenzierungspunkte 2a bleiben räumlich konstant, da sich nur das kaudal gelegene Kiefersegment bewegt. Die durch Verschiebung erhaltene neue Okklusionsebene 2c (Zahnkontakte) des Splintes 2 ausschließlich das verschobene neu positionierte Knochensegment betreffend wird dabei virtuell mit dem Planungsprogramm (Software) definiert und hergestellt.

Anschließend werden Verbindungsarme 2b zwischen Verbindungspunkten 2d und den Referenzierungspunkten 2a, auch Repositionsmarkierungen 2a genannt, hergestellt. An der Stelle der Repositionsmarkierungen 2a ist eine nicht näher bezeichnete Anhaftungsfläche mittels Software berechnet und vorgesehen, welche am Knochen aufliegt. In dieser kann ein Bohrloch vorgesehen sein, wobei dann durch Einsatz eines Befestigungsmittels eine Befestigung des nach diesen virtuellen Daten erzeugten Splint-Formteiles erfolgen kann. Als Befestigungsmittel kann insbesondere eine Halteschraube dienen. Das Bohrloch weist dabei vorzugsweise den Durchmesser des Befestigungsmittels auf. Zwischen den Verbindungsarmen 2b können wiederum Stabilisierungsverstrebungen 2e vorgesehen sein. Das segmentierte und mobile Knochensegment kann mit Halteschrauben nach Herstellung als Formteil chirurgisch am Knochen fixiert werden und definiert somit exakt die gewünschte räumliche Position des Knochensegmentes. Intraoperativ wird der Oberkiefer vom Chirurgen dann nach dem gängigen chirurgischen Konzept mit Osteosyntheseplatten in der neuen Position fixiert.

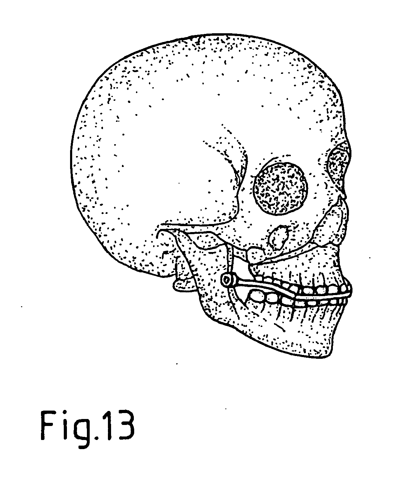

In einem dritten Schritt wird ein dritter Splint 3 (Endsplint) zur Okklusionssicherung und Reposition des Kiefergelenkes angefertigt. Entscheides Merkmal des Entsplintes 3 (siehe Fig. 4 und 13) sind Verbindungsarme 3b, die an einer Okklusionsebene 3c entsprechend der Okklusionsebene 2c des zweiten Splintes angreifen an Verbindungspunkten 3d und diese mit den Referenzierungspunkten 3a entsprechend Repositionsmarkierungen verbinden. Der Splint 3 ergänzt durch die eine Repositionsmarkierung 3a den Splint 2, so dass durch den Splint 2 und 3 die für die räumliche Lage benötigten mindestens drei Referenzierungspunkte, bezogen auf jede Seite eines Kiefers, gegeben sind.

Im Gegensatz zu Splint 2 umfasst die Okklusionsebene 3c eine durch Software berechenbare Endposition der verschobenen Knochensegmente beziehungsweise zahntragenden Knochensegmente und stellt damit die Endposition dar. Die Okklusionsebene 3c entspricht somit nicht der Okklusionsebene 2c des Splintes 2. Durch die Verbindungsarme 3b, angeordnet auf jeder Seite des Kiefers, und ausgebildete Anhaftungsflächen am Knochensegment, welches die Repositionsmarkierung 3a umfasst und am Kiefergelenkfortsatz angeordnet ist, kann die ursprüngliche, präoperative Kiefergelenksbeziehung wieder hergestellt werden. Dies erfolgt nach Herstellung des Formteiles mittels dem erfindungsgemäßen Verfahren aufgrund der vorher ermittelten virtuellen Daten dergestalt, dass mit einer Halteschraube als Haltemittel das distale, hinter der Osteotomielinie liegende Knochenstück des Gelenkfortsatzes mit dem dort definierten Referenzierungspunkt 3a mit einem Bohrloch als Befestigungsmittel gefasst wird und in die ursprüngliche präoperative Position gebracht wird. Das anteriore zahntragende Knochensegment wird durch die Okklusionsebene in seiner Lage stabilisiert.

Nun können die mobilen Knochenteile in üblicher Art und Weise mit Osteosyntheseplatten stabilisiert werden.

## Patentansprüche

1. Verfahren zur Herstellung von mindestens einem Operationssplint (1; 2; 3) unter Verwendung einer Software, umfassend die Schritte
- Erstellung einer Abbildung von Knochen eines Schädels einschließlich Kiefers aus dreidimensionalen Daten;
- Versehung der Abbildung mit mindestens drei Referenzierungspunkten (1a; 2a; 3a) an körpereigenen definierten Schädelteilen und/oder in frei wählbaren Bereichen mindestens eines ersten Knochensegmentes oberhalb bzw. gegenüber einer Osteotomielinie eines zu bewegenden Knochensegmentes;
- räumliche Ausrichtung eines zweiten, segmentierten Knochensegmentes in einer gewünschten Position;
- Versehung einer Okklusionsplatte (1c) mit mindestens einem Verbindungspunkt (1e; 2d; 3d); **dadurch gekennzeichnet, dass** das Verfahren die folgenden Schritte umfasst:
- Verbindung des Verbindungspunktes mit mindestens einem Referenzierungspunkt (1a; 2a; 3a) durch Ausbildung mindestens eines Verbindungsarmes (1d; 2b; 3b) zur Erzeugung eines virtuellen Splintes;
- Herstellung eines Splintes (1; 2; 3) auf Grundlage der mit der Software ermittelten Daten des virtuellen Splintes.

2. Verfahren gemäß Anspruch 1, **dadurch gekennzeichnet, dass** vor oder nach der Versehung der Abbildung mit mindestens drei Referenzierungspunkten (1e; 2d; 3d) eine Segmentierung des zu verschiebenden zweiten Knochensegmentes vorgenommen wird.

3. Verfahren gemäß einem der vorhergehenden Ansprüche, **dadurch gekennzeichnet, dass** mindestens eine stabilisierende Verstrebung (1f; 2e) zwischen den Verbindungsarmen (1d; 2b) erzeugt wird.

4. Verfahren gemäß einem der vorhergehenden Ansprüche, **dadurch gekennzeichnet, dass** der Splint (1; 2; 3) mit mindestens einer Markierung für eine Osteotomielinie versehen wird.

5. Verfahren gemäß einem vorhergehenden Ansprüche, **dadurch gekennzeichnet, dass** die Verbindung mindestens eines Referenzierungspunkes (1a; 2a; 3a) mit mindestens einem Verbindungspunkt dergestalt erfolgt, dass jeweils räumlich benachbarte Referenzierungs- und Verbindungspunkte (1e; 2d; 3d) miteinander verbunden werden.

6. Verfahren gemäß einem der vorhergehenden Ansprüche, **dadurch gekennzeichnet, dass** mindestens ein Operationssplint als Referenzierungssplint und/oder als Positionierungs- und/oder Repositionierungssplint erzeugt wird.

7. Verfahren gemäß einem der vorhergehenden Ansprüche, **dadurch gekennzeichnet, dass** die räumliche Ausrichtung des zweiten Knochensegmentes mittels der Software berechnet und dementsprechend Verbindungspunkte für die Okklusionsplatte ermittelt werden.

8. Verfahren gemäß einem der vorhergehenden Ansprüche, **dadurch gekennzeichnet, dass** mindestens ein dritter Operationssplint erzeugt wird, wobei eine Endposition des zweiten Knochensegmentes mittels Software bestimmt wird.

9. Verfahren gemäß einem der vorhergehenden Ansprüche, **dadurch gekennzeichnet, dass** der Positionierungs- und/oder Repositionierungssplint und der Operationssplint (1; 2; 3) an unterschiedlichen Teilbereichen des mindestens einen ersten Knochensegmentes und/oder unterschiedlichen Teilbereichen der körpereigenen definierten Schädelteile angreifbar ausgebildet werden.

10. Verfahren gemäß Anspruch 9, **dadurch gekennzeichnet, dass** mindestens einer der Teilbereiche beweglich ausgebildet ist.

11. Verfahren gemäß einem der vorhergehenden Ansprüche, **dadurch gekennzeichnet, dass** anhand eines Gipsmodelles von Zähnen ermittelte Daten digitalisiert und in die dreidimensionalen Daten der Knochen des Schädels integriert werden.

12. Verfahren gemäß Anspruch 11, **dadurch gekennzeichnet, dass** die Daten der Zähne mit denjenigen von Knochendatensätzen überlagert werden.

13. Operationssplint (1; 2; 3), hergestellt gemäß dem Verfahren nach einem der Ansprüche 1 bis 12.

## Claims

1. A method of producing at least one surgical splint (1; 2; 3) using software, comprising the steps
- creation of an image of bones of a skull including the jaw from three-dimensional data;
- provision of the image with at least three reference points (1 a; 2a; 3a), at defined bodily skull parts and/or in freely selectable regions of at least one first bone segment, above or opposite an osteotomy line of a bone segment to be moved;
- spatial orientation of a second, segmented bone segment in a desired position;
- provision of an occlusion plate (1 c) with at least one connection point (1e; 2d; 3d), **characterised in that** the method comprises the following steps:
- connection of the connection point to at least one reference point (1 a; 2a; 3a) through the formation of at least one connection arm (1d; 2b; 3b) to produce a virtual splint;
- production of a splint (1; 2; 3) on the basis of the software-determined data of the virtual splint.

2. A method according to claim 1, **characterised in that** segmentation of the second bone segment, which bone segment is to be displaced, is carried out before or after the provision of the image with at least three reference points (1e; 2d; 3d).

3. A method according to any one of the preceding claims, **characterised in that** at least one stabilising reinforcement (1f; 2e) is produced between the connection arms (1d; 2b).

4. A method according to any one of the preceding claims, **characterised in that** the splint (1; 2; 3) is provided with at least one marking for an osteotomy line.

5. A method according to any one of the preceding claims, **characterised in that** the connection of at least one reference point (1 a; 2a; 3a) to at least one connection point takes place in such a manner that in each case spatially adjacent reference and connection points (1e; 2d; 3d) are connected to one another.

6. A method according to any one of the preceding claims, **characterised in that** at least one surgical splint as a reference splint and/or as a positioning and/or repositioning splint is produced.

7. A method according to any one of the preceding claims, **characterised in that** the spatial orientation of the second bone segment is calculated by means of the software and connection points for the occlusion plate are determined accordingly.

8. A method according to any one of the preceding claims, **characterised in that** at least one third surgical splint is produced, wherein an end position of the second bone segment is determined by means of software.

9. A method according to any one of the preceding claims, **characterised in that** the positioning and/or repositioning splint and the surgical splint (1; 2; 3) are designed to be able to engage different partial regions of the at least one first bone segment and/or different partial regions of the defined bodily skull parts.

10. A method according to claim 9, **characterised in that** at least one of the partial regions is movable.

11. A method according to any one of the preceding claims, **characterised in that** data determined with the aid of a plaster-of-Paris model of teeth is digitized and integrated into the three-dimensional data of the bones of the skull.

12. A method according to claim 11, **characterised in that** the data of the teeth are overlaid with those of sets of bone data.

13. A surgical splint (1; 2; 3) produced according to the method according to any one of claims 1 to 12.

## Revendications

1. Procédé de fabrication d'au moins une gouttière chirurgicale (1 ; 2 ; 3) moyennant l'utilisation d'un logiciel, comportant les étapes :
- réalisation d'une reproduction des os d'un crâne, y compris la mâchoire, à partir de données tridimensionnelles ;
- mise en place sur la reproduction d'au moins trois points de référence (1a ; 2a ; 3a) au niveau de parties définies du crâne, propres au corps, et/ou dans des zones librement sélectionnables d'au moins un premier segment osseux au-dessus ou en face d'une ligne d'ostéotomie d'un segment osseux à déplacer ;
- orientation dans l'espace, dans une position souhaitée, d'un segment osseux segmenté ;
- mise en place d'au moins un point de liaison (le ; 2d ; 3d) sur une plaque d'occlusion (1c) ;
**caractérisé en ce que** le procédé comporte les étapes suivantes :
- jonction du point de liaison avec au moins un point de référence (1a; 2a ; 3a) par la réalisation d'au moins un bras de liaison (1d ; 2b ; 3b) en vue de générer une gouttière virtuelle ;
- fabrication d'une gouttière (1 ; 2 ; 3) sur la base des données de la gouttière virtuelle, déterminées avec le logiciel.

2. Procédé selon la revendication 1, **caractérisé en ce qu'**avant ou après la mise en place sur la reproduction d'au moins trois points de référence (1a ; 2a ; 3a), il est effectué une segmentation du deuxième segment osseux à déplacer.

3. Procédé selon l'une quelconque des revendications précédentes, **caractérisé en ce qu'**au moins un entretoisement (1f ; 2e) stabilisateur est effectué entre les bras de liaison (1d ; 2b).

4. Procédé selon l'une quelconque des revendications précédentes, **caractérisé en ce que** la gouttière (1 ; 2 ; 3) comporte au moins un marquage pour une ligne d'ostéotomie.

5. Procédé selon l'une quelconque des revendications précédentes, **caractérisé en ce que** la jonction d'au moins un point de référence (1a ; 2a ; 3a) avec au moins un point de liaison est effectuée de telle sorte que des points de référence et des points de liaison (1e; 2d ; 3d), respectivement adjacents dans l'espace, sont reliés entre eux.

6. Procédé selon l'une quelconque des revendications précédentes, **caractérisé en ce qu'**au moins une gouttière chirurgicale est réalisée en tant que gouttière de référence et/ou en tant que gouttière de positionnement et/ou en tant que gouttière de repositionnement.

7. Procédé selon l'une quelconque des revendications précédentes, **caractérisé en ce que** l'orientation dans l'espace du deuxième segment osseux est calculée au moyen du logiciel et des points de liaison sont déterminés, en conséquence, pour la plaque d'occlusion.

8. Procédé selon l'une quelconque des revendications précédentes, **caractérisé en ce qu'**au moins une troisième gouttière chirurgicale est réalisée, une position finale du deuxième segment osseux étant déterminée au moyen du logiciel.

9. Procédé selon l'une quelconque des revendications précédentes, **caractérisé en ce que** la gouttière de positionnement et/ou la gouttière de repositionnement et la gouttière chirurgicale (1 ; 2 ; 3) sont réalisées de manière à pouvoir les saisir au niveau de différentes zones partielles dudit au moins un premier segment osseux et/ou de différentes zones partielles des parties définies du crâne, propres au corps.

10. Procédé selon la revendication 9, **caractérisé en ce qu'**au moins une des zones partielles est réalisée de manière mobile.

11. Procédé selon l'une quelconque des revendications précédentes, **caractérisé en ce que** des données déterminées sont numérisées à l'appui d'un modèle en plâtre de dents et sont intégrées dans les données tridimensionnelles des os du crâne.

12. Procédé selon la revendication 11, **caractérisé en ce que** les données des dents sont superposées à celles des enregistrements de données des os.

13. Gouttière chirurgicale (1 ; 2 ; 3) fabriquée selon le procédé selon l'une quelconque des revendications 1 à 12.
